# EUROPÄISCHE PATENTSCHRIFT

(11) **EP 1 322 245 B1**
(45) Veröffentlichungstag und Bekanntmachung des Hinweises auf die Patenterteilung: **23.11.2005**
(21) Anmeldenummer: 00962155.8
(22) Anmeldetag: 04.10.2000
(51) Int. Cl.: A61B 18/14, A61N 1/18

(54) **VORRICHTUNG ZUR VERSORGUNG EINES ELEKTRO-PENS MIT ELEKTRISCHER ENERGIE**
DEVICE FOR SUPPLYING AN ELECTRO-PEN WITH ELECTRICAL ENERGY
DISPOSITIF D'ALIMENTATION EN ENERGIE ELECTRIQUE D'UN STYLO ELECTRIQUE

(43) Veröffentlichungstag der Anmeldung: 02.07.2003
(73) Patentinhaber: Synthes AG Chur, 7002 Chur (CH)
(72) Erfinder: METZGER, Roger, CH-4410 Liestal (CH)
(74) Vertreter: Lusuardi, Werther Giovanni
(86) Internationale Anmeldenummer: PCT/CH2000/000541
(87) Internationale Veröffentlichungsnummer: WO 2002/028301

(56) Entgegenhaltungen:
- DE-A- 19 713 224
- GB-A- 2 128 093
- US-A- 5 413 590
- US-A- 6 039 734
- US-A- 6 086 585
- US-A- 6 120 496

## Beschreibung

Die Erfindung betrifft eine Vorrichtung gemäss dem Oberbegriff des Anspruchs 1.

Solche Vorrichtungen gattungsgemässer Art finden insbesondere Anwendung in der Medizin sowie in der Chirurgie. Zusammen mit Elektropens werden sie zum Beispiel auch bei Operationen im Gesichts- und Handbereich verwendet.
Eine Hautbehandlungsvorrichtung mit einer netzunabhängigen Energieversorgungseinheit und einem eine Metallelektrode aufweisenden Instrument wird in der US 5,413,590 WILLIAMSON offenbart.
Die aus dem obengenannten Stand der Technik bekannte Energieversorgungseinheit für einen Elektro-Pen hat den Nachteil, dass sie konstruktionsbedingt nicht sterilisierbar ist.

Aus der DE-OS 197 13 224 A 1 ist ein Elektro-Magnet-Pen bekannt zur Therapie an Mensch und Tier. Es dient zum Einbringen von Pharmazeutika durch die menschliche und tierische Haut mit Hilfe von Magnetfeldern kleiner Intensität, ohne die Haut zu perforieren. Dieses Elektro-Pen wird mit zwei hintereinandergeschalteten Mikro-Batterien als Energieversorgungseinheit betrieben.
Ferner ist aus der US 6,039,734 GOBLE ein von Hand haltbares elektrochirurgisches Instrument bekannt, welches im Handgriff integriert einen Radiofrequez-generator und als Energieversorgungseinheit eine Batterie umfasst.

Nachteilig an diesen beiden Instrumenten ist das durch die im Handgriff integrierten Batterien erhöhte Gewicht des Instrumentes und damit dessen eingeschränkte Handlichkeit.

Der vorliegenden Erfindung liegt daher die Aufgabe zugrunde, eine Vorrichtung zur Versorgung eines Elektro-Pens mit elektrischer Energie zu schaffen, welche mobil ist und räumlich nicht an einen Netzanschluss oder an eine Konsole gebunden ist, wobei Keimfreiheit gewährleistet ist.

Diese Aufgabe wird erfindungsgemäss durch den kennzeichnenden Teil des Anspruchs 1 gelöst.

Die erfindungsgemässe Vorrichtung hat den Vorteil, dass das Netzzuleitungskabel entfällt und dadurch Mobilität gewährleistet ist. Durch das Wegfallen einer Batterie im Elektropen wird letzteres wesentlich leichter und damit handlicher. Gleichzeitig fällt eine Verbindung des Elektropens zum unsterilen Bereich weg. Ein weiterer Vorteil liegt in der direkten Bedienbarkeit aller Gerätefunktionen durch den Operateur.

Der zusammen mit der Vorrichtung verwendete Elektro-Pen hat in einer Variante zusätzlich den Vorteil, dass das Zuleitungskabel entfätft, und kein grosser und schwerer Akkumulator im Elektro-Pen selbst integriert werden muss.

Weitere vorteilhafte Ausgestaltungen der Erfindung ergeben sich aus den Unteransprüchen.

Die Konsole mit Energieversorgungseinheit ist zweckmässigerweise flüssigkeitsdicht ausgebildet, um deren Sterilisierbarkeit und Heissdampfbeständigkeit zu gewährleisten. Die Dampfsterilisierbarkeit ist gegeben bei einer Temperatur von 143 C° bei 100 % Luftfeuchtigkeit und 3 Bar Druck.

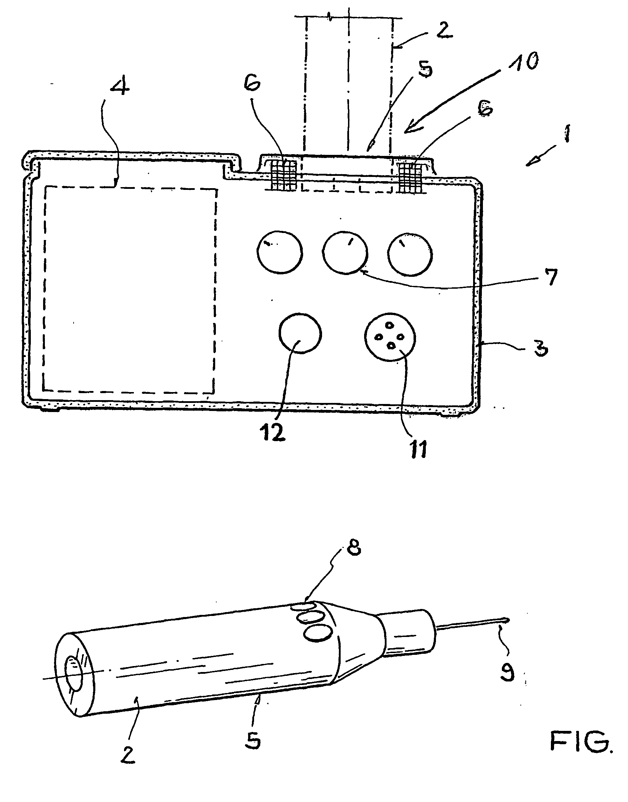

Ein Ausführungsbeispiel der Erfindung ist in der einzigen Figur dargestellt.

Es zeigt die einzige Figur die erfindungsgemässe Vorrichtung mit Konsole und Energieversorgungseinheit sowie einem Elektro-Pen.

Mit dem Bezugszeichen 1 in der einzigen Figur ist die Konsole mit Energieversorgungseinheit gekennzeichnet. Von dieser wird der Elektro-Pen 2 entweder direkt über ein Kabel, oder indirekt über eine Ladevorrichtung und kleinem Energiespeicher mit elektrischer Energie versorgt. Der Elektro-Pen 2 ist mit einer elektrischen Antriebseinheit 5 ausgestattet zum Antreiben eines Werkzeugs. In der Regel ist die elektrische Antriebseinheit 5 ein Elektromotor, doch sind auch andere Antriebseinheiten denkbar, die statt rotatorisch, translatorisch bewegt werden.

Die Konsole und Energieversorgungseinheit 1 ist erfindungsgemäss sterilisierbar. Zu diesem Zweck ist die Konsole und Energieversorgungseinheit 1 in einem flüssigkeitsdichten Gehäuse 3 eingeschlossen. Da die Konsole und Energieversorgungseinheit 1 sterilisierbar ist, kann sie im Gegensatz zu einer herkömmlichen Konsole und Energieversorgungseinheit 1 direkt vom Chirurgen bedient werden.

Die Konsole und Energieversorgungseinheit 1 ist netzunabhängig und dadurch mobil und zu diesem Zweck mit dem Akkumulator 4 als Energiequelle ausgestattet.

In einer Ausführungsvariante ist eine kleine schnellladbare elektrische Energiequelle (z.B. ein Kondensator) im Elektro-Pen 2 untergebracht, der über eine elektrische oder induktive Kopplung mit einer entsprechenden Einrichtung an der Konsole mit Energieversorgung 1 über den Akkumulator 4 aufladbar ist. Dies hat den Vorteil, dass kein grosser und schwerer Akkumulator im Elektro-Pen 2 verwendet werden muss. Zur Ladung werden entweder elektrische Kontakte oder ein Uebertrager 6 mit mindestens einer Spule in der Konsole und Energieversorgungseinheit 1 und im Elektro-Pen 2 verwendet.

Die Konsole weist eine Aufnahmehalterung 10 auf, an der das Elektro -Pen zur Ladung aufgenommen werden kann.

Durch die Netzunabhängigkeit der Konsole mit Energieversorgungseinheit 1 mittels Akkumulator 4 und das konstruktionsbedingte Weglassen von elektrischen Kabelzuführungen vom Netz zur Konsole und Energieversorgungseinheit 1 ist das ganze System nicht mehr an einen Arbeitsplatz gebunden und mobil, und kann während einer Operation von einem Platz an den anderen verschoben werden.

Mit dem Bezugszeichen 7 sind Bedienelemente der Konsole mit Energieversorgungseinheit 1 gekennzeichnet und Bezugszeichen 8 kennzeichnet die Bedienelemente des Elektro-Pens 2.

Die Konsole mit Energieversorgungseinheit 1 kann aber auch wie in der einzigen Figur dargestellt einen Kabelanschluss 12 zum Betreiben des Elekro-Pen 2 (über ein sterilisierbares Kabel) aufweisen.

Schliesslich ist die Konsole mit Energieversorgungseinheit 1 auch noch mit einem Netzanschluss 11 zur Aufladung des Akkumulators 4 versehen.

## Patentansprüche

1. Vorrichtung zur Versorgung mit elektrischer Energie eines Elektro-Pens (2) mit einer elektrischen Antriebseinheit für den Antrieb eines Werkzeugs, wobei die Vorrichtung eine Konsole mit Energieversorgungseinheit (1) umfasst, welche die elektrische Antriebseinheit des Elektro-Pens (2) mit elektrischer Energie versorgt, wobei
A) die Konsole mit Energieversorgungseinheit (1) mit einem Akkumulator (4) ausgestattet ist;
B) die elektrische Energiezuführung von der Konsole mit Energieversorgungseinheit (1) an den Elektro-Pen (2) entweder über ein sterilisierbares Kabel oder induktiv ohne elektrische Verbindung erfolgt,
**dadurch gekennzeichnet, dass**
C) die Konsole mit Energieversorgungseinheit (1) derart ausgebildet ist, dass sie dampfsterilisierbar ist.

2. Vorrichtung nach Anspruch 1, **dadurch gekennzeichnet, dass** deren elektrische und/oder elektronischen Komponenten flüssigkeitsdicht ausgebildet sind.

3. Vorrichtung nach Anspruch 1 oder 2, **dadurch gekennzeichnet, dass** die Konsole mit Energieversorgungseinheit (1) netzunabhängig und mobil ausgebildet ist.

4. Vorrichtung nach einem der Ansprüche 1 bis 4, **dadurch gekennzeichnet, dass** die Konsole mit Energieversorgungseinheit (1) einen Netzanschluss (11) zur Aufladung des Akkumulators (4) aufweist.

5. Vorrichtung nach einem der Ansprüche 1, bis 4, **dadurch gekennzeichnet, dass** die Konsole mit Energieversorgungseinheit (1) einen elektrischen Kabelanschluss (12) zur Verbindung mittels eines sterilisierbaren Kabels an den Elektro-Pen (2) aufweist.

6. Vorrichtung nach einem der Ansprüche 1 bis 5, **dadurch gekennzeichnet, dass** die Konsole und Energieversorgungseinheit (1) eine Aufnahmehalterung (10) aufweist, an welcher das Elektro-Pen (2) zur Ladung aufgenommen werden kann und wo lokal die induktive oder elektrische Kopplung zwischen Konsole und Energieversorgungseinheit (1) und Elektro-Pen (2) herstellbar ist.

7. Vorrichtung nach einem der Ansprüche 1 bis 6, **dadurch gekennzeichnet, dass** entweder ein sterilisierbarer Akku oder ein nichtsterilisierbarer Akku mit Hilfe einer Sterilabdeckung, steril in die Konsole und Energieversorgungseinheit (1) einführbar ist.

8. Vorrichtung nach einem der Ansprüche 1 bis 7, **dadurch gekennzeichnet, dass** zur Ladung des Akkumulators (4) ein Schaltnetzteil verwendet wird.

9. Vorrichtung nach einem der Ansprüche 1 bis 8, **dadurch gekennzeichnet, dass** die Vorrichtung einen Elektro-Pen (2) umfasst und dass im Elektro -Pen (2) ein elektrischer Energiespeicher, vorzugsweise ein Kondensator oder ein Akkumulator, angebracht ist, der über ein sterilisierbares Kabel oder eine induktive Kopplung mit einer entsprechenden Einrichtung an der Konsole mit Energieversorgungseinheit (1) der Vorrichtung über deren Akkumulator (4) aufladbar ist.

10. Vorrichtung nach einem der Ansprüche 1 bis 8 mit einem Elektro-Pen (2) nach Anspruch 9, **dadurch gekennzeichnet, dass** zur induktiven Kopplung ein oder mehrere übertrager (6) mit mindestens je einer Spule in der Konsole mit Energieversorgungseinheit (1) der Vorrichtung und im Elektro-Pen (2) vorgesehen sind.

11. Vorrichtung nach einem der Ansprüche 1 bis 8 mit einem Elektro-Pen (2) nach Anspruch 10, **dadurch gekennzeichnet, dass** zur elektrischen Kopplung ein oder mehrere übertrager (6) mit mindestens zwei Kontakten in der Konsole mit Energieversorgungseinheit (1) der Vorrichtung und im Elektro-Pen (2) vorgesehen sind.

## Claims

1. A device for supplying electrical energy to an electro-pen (2) having an electrical drive unit for driving a tool, whereby the device comprises a console with an energy supply unit (1) for supplying the electrical drive unit of the electro-pen (2) with electrical energy, whereby
A) the console with an energy supply unit (1) is provided with an accumulator (4);
B) the carrying of electrical energy from the console with an energy supply unit (1) to the electro-pen (2) is effected either via a sterilizable cable or inductively without electrical connection,
**characterized in that**
C) the console with an energy supply unit (1) is configured to be sterilizable by steam.

2. The device according to claim 1, wherein its electric and/or electronical components are configured liquid-tight.

3. The device according to claim 1 or 2, wherein the console with an energy supply unit (1) is configured independently of a network and mobile.

4. The device according to one of the claims 1 to 3, wherein the console with an energy supply unit (1) includes a network connector (11) for charging the accumulator (4).

5. The device according to one of the claims 1 to 4, wherein the console with an energy supply unit (1) includes an electrical cable coupling (12) for being connected to the electro-pen (2) by means of a sterilizable cable.

6. The device according to one of the claims 1 to 5, wherein the console with an energy supply unit (1) is provided with a holder (10) for receiving the electro-pen (2) in order to be charged and where the inductive or electrical coupling between the console with an energy supply unit (1) and the electro-pen (2) may be established locally.

7. The device according to one of the claims 1 to 6, wherein either a sterilizable accumulator or a non-sterilizable accumulator with a covering is sterilely introducable into the console with an energy supply unit (1).

8. The device according to one of the claims 1 to 7, wherein a switched-mode power supply for charging the accumulator (4) is used.

9. The device according to one of the claims 1 to 8, wherein the device comprises an electro-pen (2) and that the electro-pen (2) comprises an electrical energy storage device, preferably a condenser or an accumulator which is chargeable via the accumulator (4) by means of a sterilizable cable or an inductive coupling with a respective device at the console with an energy supply unit (1).

10. The device according to one of the claims 1 to 8 with an electro-pen (2) according to claim 9, wherein for the inductive coupling the console with an energy supply unit (1) of the device and the electro-pen (2) are provided with one or more transmitters (6) with at least one coil each.

11. The device according to one of the claims 1 to 8 with an electro-pen (2) according to claim 10, wherein for the electrical coupling the console with an energy supply unit (1) of the device and the electro-pen (2) are provided with one or more transmitters (6) with at least two contacts.

## Revendications

1. Dispositif permettant d'alimenter en énergie électrique un stylo électrique (2) comportant une unité d'entraînement électrique destinée à l'entraînement d'un outil, le dispositif comprenant une console comportant une unité d'alimentation en énergie (1) qui alimente en énergie électrique l'unité d'entraînement électrique du stylo électrique (2),
A) la console comportant l'unité d'alimentation en énergie (1) étant équipée d'un accumulateur (4);
B) l'amenée de l'énergie électrique depuis la console comportant l'unité d'alimentation en énergie (1) jusqu'au stylo électrique (2) étant réalisée soit par un câble stérilisable, soit de manière inductive, sans connexion électrique,
**caractérisé en ce que**
C) la console comportant l'unité d'alimentation en énergie (1) est réalisée de manière à pouvoir être stérilisée à la vapeur.

2. Dispositif selon la revendication 1, **caractérisé en ce que** ses composants électriques et/ou électroniques sont réalisés de manière à être étanches aux liquides.

3. Dispositif selon la revendication 1 ou 2, **caractérisé en ce que** la console comportant l'unité d'alimentation en énergie (1) est réalisée de manière à être indépendante du secteur et de manière à être mobile.

4. Dispositif selon l'une des revendications 1 à 4, **caractérisé en ce que** la console comportant l'unité d'alimentation en énergie (1) présente un branchement secteur (11) permettant le chargement de l'accumulateur (4).

5. Dispositif selon l'une des revendications 1 à 4, **caractérisé en ce que** la console comportant l'unité d'alimentation en énergie (1) présente une connexion de câble électrique (12) destinée à être reliée au stylo électrique (2) au moyen d'un câble stérilisable.

6. Dispositif selon l'une des revendications 1 à 5, **caractérisé en ce que** la console comportant l'unité d'alimentation en énergie (1) présente un support de positionnement (10) permettant de recevoir le stylo électrique (2) en vue du chargement et où il est possible d'établir le couplage inductif ou électrique entre la console comportant l'unité d'alimentation en énergie (1) et le stylo électrique (2).

7. Dispositif selon l'une des revendications 1 à 6, **caractérisé en ce qu'**il est possible d'insérer de manière stérile dans la console comportant l'unité d'alimentation en énergie (1) soit un accumulateur stérilisable, soit, à l'aide d'une couverture stérile, un accumulateur non-stérilisable.

8. Dispositif selon l'une des revendications 1 à 7, **caractérisé en ce qu'**un bloc d'alimentation secteur est utilisé pour le chargement de l'accumulateur (4).

9. Dispositif selon l'une des revendications 1 à 8, **caractérisé en ce que** le dispositif comprend un stylo électrique (2) et **en ce que** dans ledit stylo électrique (2) est intégré un accumulateur d'énergie électrique, de préférence un condensateur ou un accumulateur, qu'il est possible de charger à partir de l'accumulateur (4) dudit dispositif, et ce par l'intermédiaire d'un câble stérilisable ou d'un couplage inductif avec un équipement correspondant prévu sur la console comportant l'unité d'alimentation en énergie (1) du dispositif.

10. Dispositif selon l'une des revendications 1 à 8 comportant un stylo électrique (2) selon la revendication 9, **caractérisé en ce qu'**il est prévu pour le couplage inductif un ou plusieurs transformateurs (6) comportant respectivement au moins une bobine dans la console comportant l'unité d'alimentation en énergie (1) du dispositif et dans le stylo électrique (2).

11. Dispositif selon l'une des revendications 1 à 8 comportant un stylo électrique (2) selon la revendication 10, **caractérisé en ce qu'**il est prévu pour le couplage électrique un ou plusieurs transformateurs (6) comportant respectivement au moins deux contacts dans la console comportant l'unité d'alimentation en énergie (1) du dispositif et dans le stylo électrique (2).
